# EUROPEAN PATENT SPECIFICATION

(11) **EP 1 518 118 B1**
(45) Date of publication and mention of the grant of the patent: **03.10.2007**
(21) Application number: 03722820.2
(22) Date of filing: 07.05.2003
(51) Int. Cl.: G01N 33/52

(54) **SAMPLE COLLECTION DEVICE COMPRISING A HYDROPHILIC MEMBRANE FOR SEPARATING PARTICULATE MATERIAL IN THE SAMPLE**
BEHÄLTER ZUR AUFNAHME VON PROBEN, DER EINE HYDROPHILE MEMBRAN ZUR ABTRENNUNG VON PARTIKELN ENTHÄLT
DISPOSITIF DE PRELEVEMENT D'ECHANTILLONS COMPORTANT UNE MEMBRANE HYDROPHILE POUR LA SEPARATION DE MATERIAUX PARTICULAIRES DANS UN ECHANTILLON

(30) Priority: 08.05.2002 GB 0210460; 24.07.2002 US 398013 P
(43) Date of publication of application: 30.03.2005
(73) Proprietor: Yorktest Laboratories Limited, York, YO10 5DQ (GB)
(72) Inventor: RHODES, Christopher, Bruce, Osbaldwick, York YO19 5US (GB)
(74) Representative: Taylor, Kate Laura
(86) International application number: PCT/GB2003/001912
(87) International publication number: WO 2003/095969

(56) References cited:
- EP-A- 0 336 483
- EP-A- 0 926 484
- US-A- 5 306 623
- US-A- 6 020 206
- US-A1- 2002 001 846

## Description

The invention relates to a device for the rapid and volumetric collection of body fluids upon which diagnostic tests are conducted.

The preparation and/or sampling of bodily fluids, such as blood, is often a prerequisite for many assays conducted on such samples. Typically, separation methods involve chromatographic separation of particulate material from soluble material in a sample. This can often also include additional chromatographic steps to further purify the desired components from a sample.

In blood collection procedures samples of whole blood are typically collected from a patient by venous puncture through a cannula or needle attached to a syringe or collection tube. The samples are then shipped to the laboratory where the samples are processed for analysis.

A number of techniques involving the analysis of biomolecules (e.g. analytes, proteins, amino acids, DNA, RNA, intermediary metabolites), in blood samples are known. These range from basic wet chemistry methods which simply indicate the presence or absence of the biomolecule to relatively complex methods which involve sampling followed by separation, identification and quantitation of biomolecules which maybe present at very low levels. These methods typically involve the separation of fluid components using electrophoretic or chromatographic techniques followed by detection of the biomolecule(s). Examples of these techniques are disclosed in US 3,637,489 which describes a process for the separation of blood components using a technique referred to as steric chromatography, also referred to as gel chromatography, exclusion chromatography or gel permeation chromatography. The method uses a porous substance into which the blood sample permeates at different rates dependent on the size of the components. A similar methodology is employed in US 3,657,116 wherein separation of blood components from blood plasma or serum is achieved using a chromatographic column filled with porous glass particles. What is apparent is that these separation techniques take time to perform and involve a number of steps. Inevitably this means there is considerable delay between sample collection and the completion of the assay. It is often the case that the biomolecules which are to be detected are degraded or processed by contaminating cells and/or enzymes.

It would be desirable to provide a method which reduces the number of steps used in the sample isolation procedure thereby minimising loss of trace components in said sample as a consequence of degradation.

Moreover, it would also be desirable to provide a method of sample collection which is volumetric and reduces the likelihood of an insufficient sample being obtained from a patient. Furthermore it is becoming common for patients to provide samples for testing which are obtained in the home therefore removing the need for the patient to visit a doctors surgery or hospital. Blood samples are obtained by the patient by simply pricking the surface of a finger or thumb. In this case it is important that an adequate sample is taken to ensure a reliable test is provided.

Blood is a dynamic system, and as such its components/metabolites are continually being turned over, even after the samples have been removed from the body. Therefore a further technical problem that needs to be addressed in order that an accurate indication of the concentration of a metabolite in blood can be made is the prevention of this metabolite breakdown. An example of a metabolite that has a very short half-life, (223±45min, Guttormsen *et al.,* 1993) in blood is homocysteine.

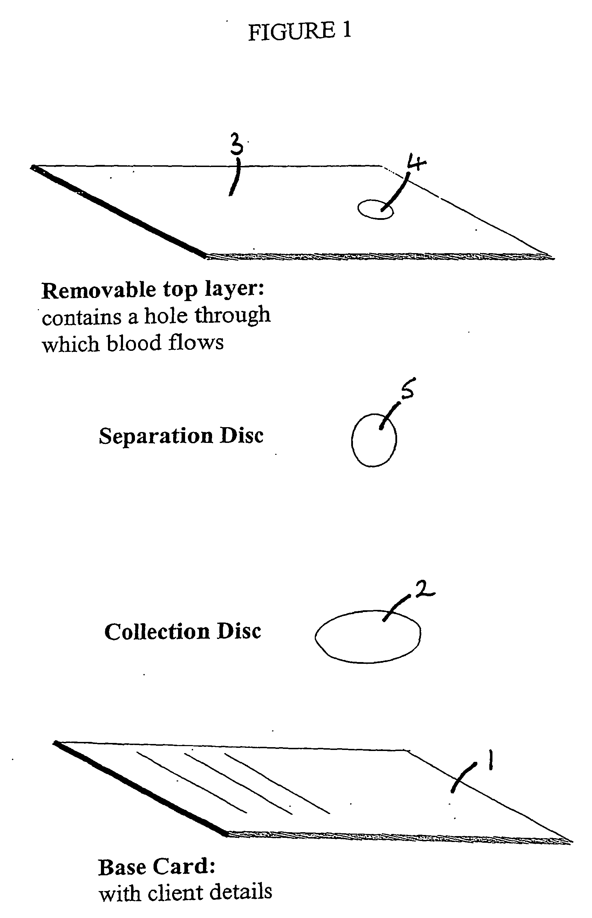

Homocysteine is an intermediary amino acid of methionine metabolism, produced when methionine is metabolized to cysteine. Generally, homocysteine produced in the body is rapidly metabolized equally by one of two routes; (1) trans-sulfuration pathway which degrades homeocysteine into cysteine and then taurine (2) remethylation pathway which regenerates methionine (see Fig. 1). The concentration of the oxidised form of homocysteine, referred to as homeocystine in the living body, under normal conditions, is negligible.

Homeocysteine levels in biological fluids, such as blood, may be of clinical significance in a number of situations as homocysteine plays an important part in a complex set of pathways which make up sulphydryl amino acid metabolism, and its accumulation may be indicative of a various disorders occurring in these pathways, including in particular inborn errors of metabolism. Thus for example, homocystinuria, characterised by an abnormal build-up of homocysteine in the urine, is known to be a disorder of amino acid metabolism resulting from deficiencies in the enzymes cystathionine beta-synthetase, cystathionine gamma-lyase or methyltetrahydrofolic acid methyltransferase.

A typical adult has an average homocysteine level of 5-10 micromoles per litre. Elevated levels of homocysteine in the blood have also be correlated with the development of atherosclerosis (Chai & Abrams, 2001) and even moderate homocysteinemia is regarded as a risk factor for cardiac and vascular diseases. An elevation of homocysteine levels by 5 micromoles per litre has also been shown to increase the risk of developing Alzheimers by 40%, with individuals having levels above 14 micromoles per litre having twice the risk of developing the disease (Seshadri et al., 2002). In addition elevated homocysteine levels have been implicated in a number of other clinical conditions, including neural tube defects, spontaneous abortion, placental abruption, low birth weight, renal failure, rheumatoid arthritis, alcoholism, osteoporosis, neuropsychiatric disorders, non-insulin-dependent diabetes and other diabetic complications.

Thus, the accurate measurement of homocysteine in blood is of great importance in the diagnosis and treatment of disease since the difference between an at risk patient and the normal level of homocysteine is marginal.

A problem associated with measuring the concentration of homocysteine in the blood is that the red blood cells contain the enzymes involved in homocysteine metabolism, e.g. cystathionine beta-synthetase and methyltetrahydrofolic acid methyltransferase, which are continually metabolising homocysteine, thereby not allowing an accurate measurement of homocysteine concentration.

Cystathione beta-synthetase (L-serine hydrolase; EC 4.2.1.22) is a unique heme enzyme that catalyzes a PLP-dependent condensation of serine and homocysteine to give cystathionine. The enzyme can be inhibited by N-alpha-p-tosyl-L-lysine chloromethyl ketone, antipain and leupeptin.

Cystathione gamma-lyase (E.C. 4.4.1.1) converts cystathionine to cysteine. It has been shown to be inhibited by the mechanism-based inhibitors; proargylglycine, trifluoroalanine and aminoethoxyvinylglycine.

Methylene tetrahydrofolate reductase is a flavoprotein that regulates the supply of one carbon units that are used to methylate homocysteine to form methionine, by catalyzing the conversion of methylenetetrahydrofolate to methyltetrahydrofolate, the methyl donor in the remethylation of homocysteine to methionine.

It would be desirable to provide a sample collection device which ensures that a sufficient sample is obtained and that the sample thus obtained provides a reliable measurement of a biomolecule in said sample. An example of such a device is disclosed in EP0336483, which is incorporated by reference. The device comprises two parts; an upper part includes a hydrophilic microporous membrane or composite of membranes which functions as a separating means for the separation of particulate materials from body fluid upon which a bioassay is conducted. A lower part includes a collecting membrane which has a defined pore volume for the collection of a defined volume of said fluid. A hydrophilic separating membrane suitable for this purpose is disclosed in EP0261734, which is incorporated by reference, which comprises a hydrophilic polymer (e.g. polyvinyl pyrrolidine) fixed to a hydrophobic polymer (e.g. polysulphone, polyether sulphone, polyetherimide). The separator membrane is asymmetric having larger pores at the sample contact surface which function to retain particulate material allowing fluid to pass through to the collecting membrane/pad.

The present invention has adapted the device disclosed in EP0336483 by the provision of indicator means which allows the patient obtaining a sample determining when an adequate sample is obtained. The device has also been adapted by the provision of means to reduce sample degradation during preparation to provide a more reliable test result.

According to an aspect of the invention there is provided a body fluid sample collection device comprising; a first substrate adapted for sample collection; a second substrate comprising at least one aperture wherein said second substrate co-operates with said first substrate to facilitate sample collection; a hydrophilic membrane which is associated with at least one substrate to provide a semi-permeable barrier to separate said fluid sample to be tested from particulate material contained in said sample wherein said first substrate is provided with indicator means which allows the determination of when a sufficient sample is obtained.

In a preferred embodiment of the invention said body fluid sample is obtained from the group consisting of; blood, urine, semen, lymph fluid, saliva, cerebral spinal fluid, synovial fluid, tears, sweat or bone marrow.

In a further preferred embodiment of the invention said sample is obtained from blood.

In a preferred embodiment of the invention said first substrate is adapted by the provision of a collection pad. Preferably said pad is mounted upon said substrate.

In a further preferred embodiment of the invention said pad is separable from said substrate. Preferably said pad is an adhesive pad.

The pad according to the invention is suitably dimensioned for the collection of a sufficient sample upon which a test can be conducted. It is particularly important that when the collection device is used in the home, without medical assistance, that the patient obtains a sufficient sample of, for example blood, to allow the technical staff to conduct the test and provide a reliable measurement of, for example, homocysteine.

In a further preferred embodiment of the invention said pad is dimensioned to ensure an adequate sample is collected. In a preferred embodiment of the invention said pad is dimensioned to enable to collection of at least about 30µl of sample. Preferably about 35 µl - 50µl of sample.

In a preferred embodiment of the invention said pad comprises an indicator. In a preferred embodiment of the invention said indicator is selected from the group consisting of; a colorimetric label; a fluorimetric label; a stain or dye.

The provision of a collection pad with an indicator ensures that a sufficient sample is obtained upon which a test can be conducted. Typically said indicator is a pH sensitive dye or stain.

In a further preferred embodiment of the invention said pad comprises at least one reducing agent.

In a further preferred embodiment of the invention said collection pad comprises at least on enzyme inhibitor.

Preferably said pad comprises an inhibitor which acts on enzymes involved in homocysteine metabolism.

In a further preferred embodiment of the invention said inhibitor acts on at least one enzyme selected from the group consisting of; cystathione beta-synthetase, cystathione gamma-lyase, methylene or tetrahydrofolate reductase.

Preferably said inhibitor is selected from the group consisting of; N-alpha-p-tosyl-L-lysine, chloromethyl ketone, antipain, leupeptin, proargylglycine, trifluoroalanine or aminoethoxyvinylglycine.

In a further preferred embodiment of the invention said membrane is in fluid connection with said collection pad.

In a preferred embodiment of the invention said membrane is placed between said substrates. Preferably said membrane is attached to at least one substrate.

In a further preferred embodiment of the invention said membrane comprises at least one reducing agent as hereindisclosed.

In a further preferred embodiment of the invention said membrane comprises at least on enzyme inhibitor as hereindisclosed.

According to a further aspect of the invention there is provided a kit comprising: a device according to the invention; a storage container for said device; a skin puncture device; and antiseptic wipes and/or adhesive dressing.

According to a further aspect of the invention there is provided a method for the collection and testing of a body fluid sample comprising the steps of:
i) providing a body fluid sample to be tested;
ii) applying the sample to the device according to the invention ; and
iii) processing said sample to determine the presence of at least one biomolecule.

In a preferred method of the invention said body fluid sample is blood.

In a further preferred method of the invention said biomolecule is homocysteine or a intermediate of homocysteine.

An embodiment of the invention will now be described by example only and with reference to the following drawing;

Figure 1 is an exploded diagrammatic representation of the sample collection device.

### Sample Collection Device

Membranes (separator and collecting pad) were purchased from Spectral Diagnostics Inc. (135-2 The West Mall Toronto, Ontaria, Canada, M9C 1C2).

Figure 1 illustrates an embodiment of the device for the separation of a body fluid, in particular plasma, from whole blood.

The device is provided with a first substrate (1) that is preferably manufactured as a rectangle from card and which forms the base of the device and is impermeable to the sample. The base card provides an area on which the patient can enter his or her personal details to allow the technician or clinician to allocate a test result to a patient.

A collection pad (2) is adhered to the upper surface of the first substrate (1). The shape of the collection pad may be circular, square, rectangular etc, but in this embodiment it is circular. The collection pad (2) is a structure which has a defined pore volume for sample collection. The adhesive pad allows ease of handling by technicians conducting an assay and, after the sample has been applied and dried to the collection pad, can be simply removed from the first substrate with suitable means (e.g. tweezers) and processed. Alternatively, a part the first substrate can be adapted for sample collection which is removed or punched from the substrate after sample application.

The collection pad (2) can be impregnated with an indicator to enable the user to determine when an adequate sample is obtained. In one example, a small dye spot in the centre of the collection pad. When the plasma migrates across the collection pad it is discoloured and the operator will see the dye carried in the solvent front of the plasma through a window provided in a second substrate (3). A second way of achieving a saturation guide is to soak the edge of the collection pad in a pH indicator, that is acidified. When the plasma reaches that point the indicator will change colour. This then shows that the disk is saturated. This happens due to the fact that plasma has a pH of 7.4, therefore changing the conditions on the collection card from acidic to neutral.

The pad optionally includes a reducing agent and/or enzyme inhibitor. A reducing agent is required when testing for homocysteine in plasma samples. Homocysteine occurs in plasma as the free thiol (trace amounts), its symmetrical disulphide (homocystine) and asymmetrical disulphides exists as conjugates with plasma proteins via disulphide linkages. The bulk of homocysteine therefore exists complexed with plasma protein making it inaccessable to assay. Samples therefore need to be reduced prior to assay to ensure a reliable measurement is made. Examples of reducing agents which may be utilised include sodium borohydride, n-butylphosphine. The addition of a reducing agent either to the sample to be added to the device or to the membrane (5) or collection pad (2) provides a means to release all homocysteine in a sample to facilitate reliable testing.

The sample and/or device may also be provided with an enzyme inhibitor to reduce the loss of the biomolecule to be detected through degradation. The inhibitor may be incorporated into the sample and/or membrane and/or collection pad. When testing for homocysteine the use of inhibitors of enzymes involved in the metabolism of homocysteine is particularly desirablen (e.g. N-alpha-p-tosyl-L-lysine, chloromethyl ketone, antipain, leupeptin, proargylglycine, trifluoroalanine or aminoethoxyvinylglycine). In addition it is also desirable to maintain cell (e.g.red blood cell) intergrity to reduce the release of degradative enzymes into a sample. It is therefore envisaged that osmotic agents (e.g. glycerol, polyethylene glycol, sucrose) are added to maintain cell membrane integrity.

Preferably the second substrate (3) is peelable and overlays the first substrate (1), thereby forming the upper layer of the device. This second substrate (3) is provided with an aperture (4) that is situated directly above and is approximately the same dimension as the collection pad (2) or region of the first substrate adapted for receiving said sample. In this preferred embodiment the aperture (4) is circular.

A membrane (5) is adhered to the underside of the second substrate (3) and is positioned so that it is aligned with the aperture (4) and collection pad (2), being directly below the aperture (4) and directly above the collection pad (2), so that the membrane (5) and the collection pad (2) are in contact with each other.

The membrane (5) is a hydrophilic microporous membrane consisting of a hydrophobic polymer and hydrophilic polymer, preferably the hydrophobic polymer is represented by polysulphone, polyether sulphone or polyetherimide, whereas the hydrophilic polymer is polyvinyl pyrrolidine. The membrane (5) is characterised by a defined pore size and porosity and is preferably asymmetrical, wherein the average pore size at one end of the membrane (5) is larger than the pore size at the opposite end of the membrane (5).

The device works by placing a small quantity of blood onto the device in a designated area. Typically 35 to 50 µl per area. Each device requires 70 to 100 µl of whole blood. The device works by capillary action. The blood soaks into the top membrane and migrates through to the collection card. The action works due to the reducing pore size of the membrane through the membrane structure.

The upper layers prevent the red blood cells from migrating to the lower membrane due to the small pore sizes. Plasma being smaller than the red blood cells it passes through the pores and onto the collection pad. The plasma then disperses over the entire collection pad. The device is left for 2 minutes to ensure complete saturation of the collection pad.

Whole blood is applied to the side of the membrane with the large pores. While the blood migrates throw the membrane, all the blood cells become trapped in the maze of pores owing to the asymmetry of the membranes. This then leaves the plasma to migrate through to the collection membrane. The driving force for the separation process is the capillary action between separation and collection membrane. The membrane material is a hydrophilic polymer with no affinity for proteins.

In intimate contact with the collection pad is the separation membrane. In one embodiment the separation membrane is significantly smaller than the collection pad. The reasons for the reduction in size are to optimise recovery of the plasma and reduce the dead volume in the separation membrane.

The first substrate is the same dimension as the second substrate and is able to be removed easily from the second substrate. The first substrate has two apertures. One is directly above the first substrate. This is to enable the operator to apply their blood to the device. The second aperture is to allow the operator to see the edge of the collection pad. This is to enable the operator to determine when enough blood has been applied and saturated the collection pad. Therefore altering the size of the collection pad will yield different volumetric amounts of plasma.

## Claims

1. A body fluid sample collection device comprising; a first substrate adapted for sample collection; a second substrate comprising at least one aperture wherein said second substrate co-operates with said first substrate to facilitate sample collection; a hydrophilic membrane which is associated with at least one substrate to provide a semi-permeable barrier to separate said fluid sample to be tested from particulate material contained in said sample, wherein said first substrate is provided with indicator means which allows the determination of when a sufficient sample is obtained.

2. A device according to Claim 1 wherein said sample is obtained from the group consisting of; blood, urine, semen, lymph fluid, saliva, cerebral spinal fluid, synovial fluid, tears, sweat or bone marrow.

3. A device according to Claim 2 wherein said sample is obtained from blood.

4. A device according to any of Claims 1-3 said first substrate is adapted by the provision of a collection pad.

5. A device according to Claim 4 wherein said pad is mounted upon said substrate.

6. A device according to Claim 4 or 5 wherein said pad is separable from said substrate.

7. A device according to Claim 6 wherein said pad is an adhesive pad.

8. A device according to any of Claims 4-7 wherein said pad is dimensioned to ensure an adequate sample is collected.

9. A device according to Claim 8 wherein said pad is dimensioned to enable the collection of at least about a 35µl sample.

10. A device according to Claim 9 wherein said pad is dimensioned to collect between about 35µl - 50µl of sample.

11. A device according to any of Claims 4-10 wherein said pad comprises an indicator.

12. A device according to Claim 11 wherein said indicator is selected from the group consisting of; colorimetric label; fluorimetric label; a stain or dye.

13. A device according to any of Claims 4-12 wherein said pad comprises at least one reducing agent.

14. A device according to any of Claims 4-13 wherein said collection pad comprises at least on enzyme inhibitor.

15. A device according to Claim 14 wherein said collection pad comprises an inhibitor which acts on enzymes involved in homocysteine metabolism.

16. A device according to Claim 15 wherein said inhibitor acts on at least one enzyme selected from the group consisting of; cystathione beta-synthetase, cystathione gamma-lyase, methylene or tetrahydrofolate reductase.

17. A device according to Claim 16 wherein said inhibitor is selected from the group consisting of; N-alpha-p-tosyl-L-lysine, chloromethyl ketone, antipain, leupeptin, proargylglycine, trifluoroalanine or aminoethoxyvinylglycine.

18. A device according to any of Claims 1-17 wherein said membrane is in fluid connection with said collection pad.

19. A device according to Claim 18 wherein said membrane is placed between said substrates.

20. A device according to any of Claims 1-19 wherein said membrane comprises at least one reducing agent.

21. A device according to any of Claims 1-20 wherein said membrane comprises at least on enzyme inhibitor.

22. A kit comprising: a device according to any of Claims 1-21; a storage container for said device; a skin puncture device; and antiseptic wipes and/or adhesive dressing.

23. A method for the collection and testing of a body fluid sample comprising the steps of:
i) providing a body fluid sample to be tested;
ii) applying the sample to the device according to any of Claims 1-22; and
iii) processing said sample to determine the presence of at least one biomolecule.

24. A method according to Claim 23 wherein said body fluid sample is blood.

25. A method according to Claim 23 or 24 wherein said biomolecule is homocysteine or a intermediate of homocysteine.

## Patentansprüche

1. Vorrichtung zum Sammeln von Körperflüssigkeitsproben, die ein erstes Substrat, das zur Probensammlung geeignet ist, ein zweites Substrat, das mindestens eine Öffnung umfasst, wobei das zweite Substrat mit dem ersten Substrat zusammenwirkt, um die Probensammlung zu erleichtern, und eine hydrophile Membran umfasst, die mit mindestens einem Substrat verbunden ist, um eine semipermeable Barriere zu liefern, um die zu untersuchende Flüssigkeitsprobe von in der Probe enthaltenem teilchenförmigen Material zu trennen, wobei das erste Substrat mit Indikatormitteln versehen ist, die die Bestimmung des Zeitpunkts ermöglichen, wenn eine ausreichende Probe erhalten ist.

2. Vorrichtung nach Anspruch 1, bei der die Probe aus der Gruppe bestehend aus Blut, Urin, Samen, Lymphflüssigkeit, Speichel, Hirn-Rückenmarksflüssigkeit, Gelenkflüssigkeit, Tränen, Schweiß und Knochenmark erhalten wird.

3. Vorrichtung nach Anspruch 2, bei der die Probe aus Blut erhalten wird.

4. Vorrichtung nach einem der Ansprüche 1 bis 3, bei der das erste Substrat durch die Bereitstellung eines Sammelpads geeignet ist.

5. Vorrichtung nach Anspruch 4, bei der das Pad auf dem Substrat angebracht ist.

6. Vorrichtung nach Anspruch 4 oder 5, bei der das Pad von dem Substrat trennbar ist.

7. Vorrichtung nach Anspruch 6, bei der das Pad eine haftfähiges Pad ist.

8. Vorrichtung nach einem der Ansprüche 4 bis 7, bei der das Pad bemessen ist, um sicherzustellen, dass genügend Probe gesammelt wird.

9. Vorrichtung nach Anspruch 8, bei der das Pad bemessen ist, um die Sammlung von mindestens etwa 35 µl Probe zu ermöglichen.

10. Vorrichtung nach Anspruch 9, bei der das Pad bemessen ist, um etwa 35 µl bis 50 µl Probe zu sammeln.

11. Vorrichtung nach einem der Ansprüche 4 bis 10, bei der das Pad einen Indikator umfasst.

12. Vorrichtung nach Anspruch 11, bei der der Indikator ausgewählt ist aus der Gruppe bestehend aus kolorimetrischer Indizierung, fluorimetrischer Indizierung, Färbemittel und Farbstoff.

13. Vorrichtung nach einem der Ansprüche 4 bis 12, bei der das Pad mindestens ein Reduktionsmittel umfasst.

14. Vorrichtung nach einem der Ansprüche 4 bis 13, bei der das Sammelpad mindestens einen Enzyminhibitor umfasst.

15. Vorrichtung nach Anspruch 14, bei der das Sammelpad einen Inhibitor umfasst, der auf Enzyme wirkt, die im Homocysteinmetabolismus einbezogen sind.

16. Vorrichtung nach Anspruch 15, bei der der Inhibitor auf mindestens ein Enzym ausgewählt aus der Gruppe bestehend aus Cystathion-β-Synthase, Cystathion-γ-Lyase, Methylen- und Tetrahydrofolatreduktase wirkt.

17. Vorrichtung nach Anspruch 16, bei der der Inhibitor ausgewählt ist aus der Gruppe bestehend aus N-α-p-Tosyl-L-lysin, Chlormethylketon, Antipain, Leupeptin, Proargylglycin, Trifluoralanin und Aminoethoxyvinylglycin.

18. Vorrichtung nach einem der Ansprüche 1 bis 17, bei der sich die Membran in flüssiger Verbindung mit dem Sammelpad befindet.

19. Vorrichtung nach Anspruch 18, bei der die Membran zwischen den Substraten angeordnet ist.

20. Vorrichtung nach einem der Ansprüche 1 bis 19, bei der die Membran mindestens ein Reduktionsmittel umfasst.

21. Vorrichtung nach einem der Ansprüche 1 bis 20, bei der die Membran mindestens einen Enzyminhibitor umfasst.

22. Kit, der eine Vorrichtung nach einem der Ansprüche 1 bis 21, einen Aufbewahrungsbehälter für die Vorrichtung, eine Hauteinstichvorrichtung und antiseptische Tücher und/oder haftfähiges Verbandmaterial umfasst.

23. Verfahren zum Sammeln und Untersuchen einer Körperflüssigkeitsprobe, bei dem
i) eine zu untersuchende Körperflüssigkeitsprobe bereitgestellt wird,
ii) die Probe auf die Vorrichtung gemäß einem der Ansprüche 1 bis 22 angewendet wird und
iii) die Probe verarbeitet wird, um das Vorhandensein mindestens eines Biomoleküls zu bestimmen.

24. Verfahren nach Anspruch 23, bei dem die Körperflüssigkeitsprobe Blut ist.

25. Verfahren nach Anspruch 23 oder 24, bei dem das Biomolekül Homocystein oder ein Intermediat von Homocystein ist.

## Revendications

1. - Dispositif de collecte d'échantillon de fluide corporel comprenant un premier substrat adapté pour la collecte d'échantillon ; un second substrat comprenant au moins une ouverture, ledit second substrat coopérant avec ledit premier substrat pour faciliter la collecte d'échantillon ; une membrane hydrophile qui est associée à au moins un substrat pour fournir une barrière semi-perméable pour séparer ledit d'échantillon de fluide devant être testé du matériel particulaire contenu dans ledit échantillon, ledit premier substrat étant doté d'un moyen indicateur qui permet la détermination du moment où un échantillon suffisant est obtenu.

2. - Dispositif selon la revendication 1, dans lequel ledit échantillon est obtenu à partir du groupe constitué par : le sang, l'urine, le sperme, la lymphe, la salive, le liquide céphalo-rachidien, le liquide synovial, les larmes, la sueur ou la moelle osseuse.

3. - Dispositif selon la revendication 2, dans lequel ledit échantillon est obtenu à partir du sang.

4. - Dispositif selon l'une quelconque des revendications 1-3, dans lequel ledit premier substrat est adapté par la disposition d'un tampon de collecte.

5. - Dispositif selon la revendication 4, dans lequel ledit tampon est monté sur ledit substrat.

6. - Dispositif selon l'une des revendications 4 ou 5, dans lequel ledit tampon est séparable dudit substrat.

7. - Dispositif selon la revendication 6, dans lequel ledit tampon est un tampon adhésif.

8. - Dispositif selon l'une quelconque des revendications 4-7, dans lequel ledit tampon est dimensionné pour assurer qu'un échantillon suffisant est collecté.

9. - Dispositif selon la revendication 8, dans lequel ledit tampon est dimensionné pour permettre la collecte d'un échantillon d'au moins environ 35 µl.

10. - Dispositif selon la revendication 9, dans lequel ledit tampon est dimensionné pour collecter entre environ 35 µl-50 µl d'échantillon.

11. - Dispositif selon l'une quelconque des revendications 4-10, dans lequel ledit tampon comprend un indicateur.

12. - Dispositif selon la revendication 11, dans lequel ledit indicateur est choisi dans le groupe constitué par un marqueur colorimétrique, un marqueur fluorimétrique, une teinture ou un colorant.

13. - Dispositif selon l'une quelconque des revendications 4-12, dans lequel ledit tampon comprend au moins un agent réducteur.

14. - Dispositif selon l'une quelconque des revendications 4-13, dans lequel ledit tampon de collecte comprend au moins un inhibiteur enzymatique.

15. - Dispositif selon la revendication 14, dans lequel ledit tampon de collecte comprend un inhibiteur qui agit sur les enzymes mises en jeu dans le métabolisme de l'homocystéine.

16. - Dispositif selon la revendication 15, dans lequel ledit inhibiteur agit sur au moins une enzyme choisie dans le groupe constitué par la cystathione bêta-synthétase, la cystathione gamma-lyase, la méthylène ou tétrahydrofolate réductase.

17. - Dispositif selon la revendication 16, dans lequel ledit inhibiteur est choisi dans le groupe constitué par la N-alpha-p-tosyl-L-lysine, la chlorométhyl cétone, l'antipaïne, la leupeptine, la proargylglycine, la trifluoroalanine ou l'aminoéthoxyvinylglycine.

18. - Dispositif selon l'une quelconque des revendications 1-17, dans lequel ladite membrane est en connection de fluide avec ledit tampon de collecte.

19. - Dispositif selon la revendication 18, dans lequel ladite membrane est placée entre lesdits substrats.

20. - Dispositif selon l'une quelconque des revendications 1-19, dans lequel ladite membrane comprend au moins un agent réducteur.

21. - Dispositif selon l'une quelconque des revendications 1-20, dans lequel ladite membrane comprend au moins un inhibiteur enzymatique.

22. - Kit comprenant un dispositif tel que défini à l'une quelconque des revendications 1-21 ; un conteneur de stockage pour ledit dispositif ; un dispositif de piqûre de la peau ; et des éponges antiseptiques et/ou un pansement adhésif.

23. - Procédé pour la collecte et le test d'un échantillon de fluide corporel comprenant les étapes consistant à :
(i) se procurer un échantillon de fluide corporel devant être testé ;
(ii) appliquer l'échantillon au dispositif tel que défini à l'une quelconque des revendications 1-22 ; et
(iii) traiter ledit échantillon pour déterminer la présence d'au moins une biomolécule.

24. - Procédé selon la revendication 23, dans lequel ledit échantillon de fluide corporel est du sang.

25. - Procédé selon l'une des revendications 23 ou 24, dans lequel ladite biomolécule est l'homocystéine ou un intermédiaire de l'homocystéine.
